(19) Europäisches Patentamt
European Patent Office
Office européen des brevets

(11) Publication number : **0 514 216 A1**

(12) **EUROPEAN PATENT APPLICATION**

(21) Application number : **92304448.1**

(51) Int. Cl.[5] : **C07D 405/06,** A61K 31/415

(22) Date of filing : **15.05.92**

(30) Priority : **16.05.91 GB 9110635**

(43) Date of publication of application : **19.11.92 Bulletin 92/47**

(84) Designated Contracting States : **AT BE CH DE DK ES FR GB GR IT LI LU MC NL PT SE**

(71) Applicant : **GLAXO GROUP LIMITED**
**Glaxo House, Berkeley Avenue**
**Greenford, Middlesex UB6 0NN (GB)**

(72) Inventor : **Ross, Barry Clive**
**Glaxo Group Research Limited, Park Road**
**Ware, Hertfordshire SG12 0DG (GB)**
Inventor : **Middlemiss, David**
**Glaxo Group Research Limited, Park Road**
**Ware, Hertfordshire SG12 0DG (GB)**
Inventor : **Scopes, David Ian Carter**
**Glaxo Group Research Limited, Park Road**
**Ware, Hertfordshire SG12 0DG (GB)**
Inventor : **Jack, Torquil Iain MacLean**
**Glaxo Group Research Limited, Park Road**
**Ware, Hertfordshire SG12 0DG (GB)**
Inventor : **Cardwell, Kevin Stuart**
**Glaxo Group Research Limited, Park Road**
**Ware, Hertfordshire SG12 0DG (GB)**
Inventor : **Dowle, Michael Dennis**
**Glaxo Group Research Limited, Park Road**
**Ware, Hertfordshire SG12 0DG (GB)**
Inventor : **Judd, Duncan Bruce**
**Glaxo Group Research Limited, Park Road**
**Ware, Hertfordshire SG12 0DG (GB)**

(74) Representative : **James, Stephen Richard, Dr. et al**
**Glaxo Holdings plc Glaxo House Berkeley Avenue**
**Greenford, Middlesex UB6 0NN (GB)**

(54) **Antihypotensive benzofuran derivatives with N-linked 1H-imidazolyl-methyl-5-carboxamide substituents.**

(57) The invention provides compounds of the general formula (I) :

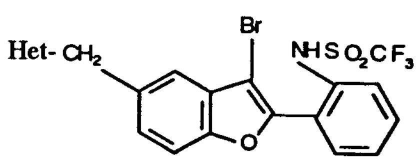

(I)

or a physiologically acceptable salt or solvate thereof in which Het represents an N-linked imidazolyl group of the formula

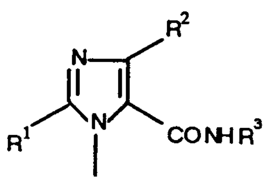

;

$R^1$ represents an ethyl or n-propyl group ;
$R^2$ represents a chlorine atom or a methyl or ethyl group ; and
$R^3$ represents a hydrogen atom or a methyl or ethyl group.

The compounds may be used in the treatment or prophylaxis of hypertension and diseases associated with cognitive disorders.

EP 0 514 216 A1

This invention relates to benzofuran derivatives, processes for their preparation and pharmaceutical compositions containing them. According to a first aspect of the invention we provide a compound of the general formula (I):

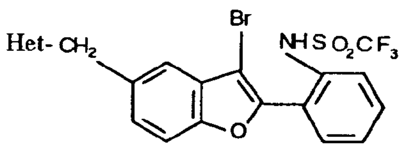

(I)

or a physiologically acceptable salt or solvate (e.g. hydrate) thereof in which Het represents an N-linked imidazolyl group of the formula

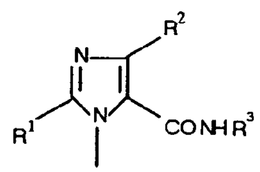

;

$R^1$ represents an ethyl or n-propyl group;
$R^2$ represents a chlorine atom or a methyl or ethyl group; and
$R^3$ represents a hydrogen atom or a methyl or ethyl group.

One advantage of the compounds of the present invention is their particularly desirable degree of bioavailability. Thus, in renal-ligated hypertensive rats, compounds of the present invention when administered both orally and intra-arterially produce falls in blood pressure which are of similar magnitude and duration. When compounds of the present invention are tested in conscious normotensive dogs, measurement of plasma concentration shows that at least 40% of an orally administered dose of the test compound is absorbed.

The invention also includes within its scope the solvates, especially the hydrates of compounds of general formula (I).

The physiologically acceptable acid addition salts of the compounds of general formula (I) may be derived from inorganic or organic acids. Examples of such salts include hydrochlorides, hydrobromides, sulphates, phosphates, benzoates, methanesulphonates or trifluoroacetates.

It will be appreciated that, for pharmaceutical use, the salts referred to above will be physiologically acceptable, but other salts may find use, for example, in the preparation of the compounds of general formula (I) and the physiologically acceptable salts thereof.

Represented by the above general formula (I) are:

1-[[3-bromo-2-[2-[[(trifluoromethyl)sulphonyl]amino]phenyl]-5-benzofuranyl]methyl]-4-chloro-2-ethyl-1H-imidazole-5-carboxamide;

1-[[3-bromo-2-[2-[[(trifluoromethyl)sulphonyl]amino]phenyl]-5-benzofuranyl]methyl]-4-chloro-2-ethyl-N-methyl-1H-imidazole-5-carboxamide;

1-[[3-bromo-2-[2-[[(trifluoromethyl)sulphonyl]amino]phenyl]-5-benzofuranyl]methyl]-4-chloro-N,2-diethyl-1H-imidazole-5-carboxamide;

1-[[3-bromo-2-[2-[[(trifluoromethyl)sulphonyl]amino]phenyl]-5-benzofuranyl]methyl]-2-ethyl-4-methyl-1H-imidazole-5-carboxamide;

1-[[3-bromo-2-[2-[[(trifluoromethyl)sulphonyl]amino]phenyl]-5-benzofuranyl]methyl]-2-ethyl-N,4-dimethyl-1H-imidazole-5-carboxamide;

1-[[3-bromo-2-[2-[[(trifluoromethyl)sulphonyl]amino]phenyl]-5-benzofuranyl]methyl]-N,2-diethyl-4-methyl-1H-imidazole-5-carboxamide;

1-[[3-bromo-2-[2-[[(trifluoromethyl)sulphonyl]amino]phenyl]-5-benzofuranyl]methyl]-4-chloro-2-propyl-1H-imidazole-5-carboxamide;

1-[[3-bromo-2-[2-[[(trifluoromethyl)sulphonyl]amino]phenyl]-5-benzofuranyl]methyl]-4-chloro-N-methyl-2-propyl-1H-imidazole-5-carboxide;

1-[[3-bromo-2-[2-[[(trifluoromethyl)sulphonyl]amino]phenyl]-5-benzofuranyl]methyl]-4-chloro-N-ethyl-2-propyl-1H-imidazole-5-carboxamide;

1-[[3-bromo-2-[2-[[(trifluoromethyl)sulphonyl]amino]phenyl]-5-benzofuranyl]methyl]-4-methyl-2-propyl-1H-imidazole-5-carboxamide;

1-[[3-bromo-2-[2-[[(trifluoromethyl)sulphonyl]amino]phenyl]-5-benzofuranyl]methyl]-N,4-dimethyl-2-propyl-1H-imidazole-5-carboxamide;
1-[[3-bromo-2-[2-[[(trifluoromethyl)sulphonyl]amino]phenyl]-5-benzofuranyl]methyl]-N-ethyl-4-methyl-2-propyl-1H-imidazole-5-carboxamide;
1-[[3-bromo-2-[2-[[(trifluoromethyl)sulphonyl]amino]phenyl]-5-benzofuranyl]methyl]-2,4-diethyl-1H-imidazole-5-carboxamide;
1-[[3-bromo-2-[2-[[(trifluoromethyl)sulphonyl]amino]phenyl]-5-benzofuranyl]methyl]-2,4-diethyl-N-methyl-1H-imidazole-5-carboxamide;
1-[[3-bromo-2-[2-[[(trifluoromethyl)sulphonyl]amino]phenyl]-5-benzofuranyl]methyl]-N,2,4-triethyl-1H-imidazole-5-carboxamide;
1-[[3-bromo-2-[2-[[(trifluoromethyl)sulphonyl]amino]phenyl]-5-benzofuranyl]methyl]-4-ethyl-2-propyl-1H-imidazole-5-carboxamide;
1-[[3-bromo-2-[2-[[(trifluoromethyl)sulphonyl]amino]phenyl]-5-benzofuranyl]methyl]-4-ethyl-N-methyl-2-propyl-1H-imidazole-5-carboxamide;
1-[[3-bromo-2-[2-[[(trifluoromethyl)sulphonyl]amino]phenyl]-5-benzofuranyl]methyl]-N,4-diethyl-2-propyl-1H-imidazole-5-carboxamide;
and physiologically acceptable salts and solvates thereof.

Particularly preferred compounds of the present invention include:
1-[[3-bromo-2-[2-[[(trifluoromethyl)sulphonyl]amino]phenyl]-5-benzofuranyl]methyl]-N,4-dimethyl-2-ethyl-1H-imidazole-5-carboxamide;
1-[[3-bromo-2-[2-[[(trifluoromethyl)sulphonyl]amino]phenyl]-5-benzofuranyl]methyl]-4-chloro-2-ethyl-1H-imidazole-5-carboxamide;
1-[[3-bromo-2-[2-[[(trifluoromethyl)sulphonyl]amino]phenyl]-5-benzofuranyl]methyl]-4-chloro-N-methyl-2-propyl-1H-imidazole-5-carboxamide;
1-[[3-bromo-2-[2-[[(trifluoromethyl)sulphonyl]amino]phenyl]-5-benzofuranyl]methyl]-4-chloro-N-ethyl-2-propyl-1H-imidazole-5-carboxamide;
1-[[3-bromo-2-[2-[[(trifluoromethyl)sulphonyl]amino]phenyl]-5-benzofuranyl]methyl]-4-chloro-2-ethyl-N-methyl-1H-imidazole-5-carboxamide;
1-[[3-bromo-2-[2-[[(trifluoromethyl)sulphonyl]amino]phenyl]-5-benzofuranyl]methyl]-2-ethyl-4-methyl-1H-imidazole-5-carboxamide;
1-[[3-bromo-2-[2-[[(trifluoromethyl)sulphonyl]amino]phenyl]-5-benzofuranyl]methyl]-N,4-dimethyl-2-propyl-1H-imidazole-5-carboxamide;
1-[[3-bromo-2-[2-[[(trifluoromethyl)sulphonyl]amino]phenyl]-5-benzofuranyl]methyl]-4-methyl-2-propyl-1H-imidazole-5-carboxamide;
and physiologically acceptable salts and solvates thereof.

Especially preferred compounds of the present invention are:
1-[[3-bromo-2-[2-[[(trifluoromethyl)sulphonyl]amino]phenyl]-5-benzofuranyl]methyl]-N,4-dimethyl-2-ethyl-1H-imidazole-5-carboxamide;
1-[[3-bromo-2-[2-[[(trifluoromethyl)sulphonyl]amino]phenyl]-5-benzofuranyl]methyl]-N-ethyl-4-methyl-2-propyl-1H-imidazole-5-carboxamide;
and physiologically acceptable salts and solvates thereof.

A preferred class of compounds of general formula (I) is that wherein $R^2$ represents a chlorine atom or a methyl group.

According to a second aspect of the present invention we provide a compound of general formula (I) or a physiologically acceptable salt or solvate thereof for use in therapy.

In particular, the compounds of the present invention may be used in the treatment or prophylaxis of hypertension (for example, essential, malignant or resistant, caused by oral contraceptives, coarctation of the aorta or renal vascular disease) and pulmonary hypertension.

The compounds of the present invention may also be used in the treatment or prophylaxis of congestive heart failure, acute or chronic heart failure, aortic or cardiac insufficiency, post-myocardial infarction, renal insufficiency and renal failure (for example, as a result of diabetic nephropathy, glomerular nephritis, scleroderma or renal crisis), proteinuria, Bartter's syndrome, secondary hyperaldosteronism, Reynaud's syndrome, cerebrovascular insufficiency, peripheral vascular disease, diabetic retinopathy, atherogenesis and for the improvement of vascular compliance.

They are also potentially useful for the treatment of cognitive disorders such as dementia (e.g. Alzheimer's disease) and other CNS disorders, such as anxiety disorders, schizophrenia, depression and alcohol or drug (e.g. cocaine) dependency.

According to a further aspect of the present invention we provide a compound of general formula (I) or a physiologically acceptable salt or solvate thereof for use in the treatment of the aforementioned diseases, especially hypertension.

According to another aspect of the present invention we provide a compound of general formula (I) or a physiologically acceptable salt or solvate thereof for the manufacture of a therapeutically thereof for the manufacture of a therapeutic agent for the treatment of the aforementioned diseases, especially hypertension.

According to a further aspect of the present invention we provide a method of treating the aforementioned diseases, especially hypertension, which method comprises administering an effective amount to a patient in need of such treatment of a compound of general formula (I) or a physiologically acceptable salt or solvate thereof.

It will be appreciated that the compounds of general formula (I) or a physiologically acceptable salt or solvate thereof may advantageously be used in conjunction with one or more other therapeutic agents, such as for example diuretics and/or different antihypertensive agents such as β-blockers, calcium channel blockers or ACE inhibitors. It is to be understood that such combination therapy constitutes a further aspect of the present invention.

It will be further appreciated that reference herein to treatment extends to prophylaxis as well as to the treatment and relief of established symptoms.

While it is possible that a compound of general formula (I) may be administered as the raw chemical it is preferable to present the active ingredient as a pharmaceutical formulation.

The compounds of general formula (I) and their physiologically acceptable salts and solvates may be formulated for administration in any convenient way, and the invention also includes within its scope pharmaceutical compositions comprising at least one compound of general formula (I) or a physiologically acceptable salt or solvate thereof adapted for use in human or veterinary medicine. Such compositions may be presented for use in conventional manner in admixture with one or more physiologically acceptable carriers or excipients. The carrier(s) must be 'acceptable' in the sense of being compatible with the other ingredients of the formulation and not deleterious to the recipient thereof.

Thus, the compounds according to the invention may be formulated for oral, buccal, parenteral or rectal administration or in a form suitable for administration by inhalation or insufflation. Oral administration is preferred.

Tablets and capsules for oral administration may contain conventional excipients such as binding agents, for example mucilage of starch or polyvinylpyrrolidone; fillers, for example, lactose, microcrystalline cellulose or maize-starch; lubricants, for example, magnesium stearate or stearic acid; disintegrants, for example, potato starch, croscarmellose sodium or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup or carboxymethyl cellulose; emulsifying agents, for example, sorbitan mono-oleate; non-aqueous vehicles (which may include edible oils), for example, propylene glycolor ethyl alcohol; and preservatives, for example, methyl or propyl p-hydroxybenzoates or sorbic acid. The compounds or their salts or esters may also be formulated as suppositories, e.g. containing conventional suppository bases such as cocoa butter or other glycerides. For buccal administration the composition may take the form of tablets or lozenges formulated in conventional manner.

It will be appreciated that both tablets and capsules may be manufactured in the form of sustained release formulations, such that they provide a controlled continuous release of the compounds according to the invention over a period of hours.

The compounds of general formula (I) and their physiologically acceptable salts and solvates may be formulated for parenteral administration by bolus injection or continuous infusion and may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoro methane, dichlorotetrafluoroethane or other suitable gas. In the case of a pressurised aerosol the dosage unit may be determined by providing a valve to deliver a metered amount.

Alternatively, for administration by inhalation or insufflation, the compounds according to the invention may take the form of a dry powder composition, for example a powder mix of the compound and a suitable powder base such as lactose or starch. The powder composition may be presented in unit dosage form in, for example,

capsules or cartridges of e.g. gelatin, or blister packs from which the powder may be administered with the aid of an inhaler or insufflator.

The pharmaceutical formulations according to the invention may also contain other active ingredients such as antimicrobial agents, or preservatives.

It will be appreciated that the amount of a compound of general formula (I) required for use in treatment will vary not only with the particular compound selected but also with the route of administration, the nature of the condition being treated and the age and condition of the patient and will ultimately be at the discretion of the attendant physician or veterinarian. In general, however, when the compositions comprise dosage units, each unit will preferably contain 5mg to 500mg, advantageously where the compounds are to be administered orally 25mg to 400mg of the active compound. The daily dosage as employed for adult human treatment will preferably range from 5mg to 3g, most preferably from 25mg to 1g which may be administered in 1 to 4 daily doses.

The compounds of the invention may be prepared by a number of processes as described below wherein the various groups are as defined for general formula (I) unless otherwise specified.

Thus, according to a further aspect of the present invention we provide a process (A) for preparing the compounds of general formula (I) which comprises treating a compound of general formula (II)

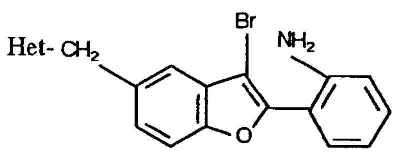

(II)

(wherein Het is as defined in general formula (I)) with trifluoromethanesulphonic anhydride or trifluoromethylsulphonyl chloride, in a suitable solvent such as a halogenated hydrocarbon, e.g. dichloromethane or chloroform optionally in the presence of a base, such as triethylamine, at a temperature between -100°C and 0°C, and preferably between -90°C and -60°C.

In another general process (B) a compound of general formula (I) may be prepared by reaction of a compound of formula (III)

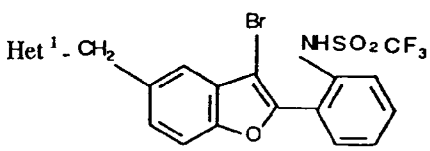

(III)

(wherein Het[1] represents a group of formula

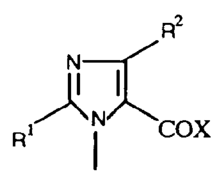

in which $R^1$ and $R^2$ are as defined in general formula (I) and X is a halogen atom (for example chlorine or bromine), or a hydroxyl or $C_{1-6}$alkoxy (for example, methoxy, ethoxy or propoxy) group) with ammonia ($R^3$ = hydrogen), methylamine ($R^3$ = methyl) or ethylamine ($R^3$ = ethyl).

Where X is a halogen atom the reaction is a Schotten-Baumann procedure, preferably being effected in the presence of a base such as aqueous sodium hydroxide or pyridine at a temperature between -20°C and 50°C, preferably between -5°C and room temperature.

Where X is a hydroxyl group the reaction may be effected under standard conditions of amide formation, preferably in the presence of a suitable coupling agent, such as N,N′-carbonyldiimidazole (CDI) or dicyclohexylcarbodiimide. The reaction is conveniently effected in a solvent such as a substituted amide e.g. dimethylformamide, an ether e.g. tetrahydrofuran, or a halogenated hydrocarbon e.g. dichloromethane at a temperature between 0° and 100°C, and conveniently at room temperature.

Where X is a $C_{1-6}$alkoxy group, the reaction may be effected in the presence of an amine such as anhydrous

methylamine in a sealed vessel at a temperature between room temperature and 100°C.

In the processes (A) and (B) described above, the compounds of general formula (I) may be obtained in the form of a salt, conveniently in the form of a physiologically acceptable salt. Where desired, such salts may be converted into the corresponding free acids or free bases using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or isopropanol.

Physiologically acceptable salts may also be prepared from other salts, including other physiologically acceptable salts, of the compounds of general formula (I), using conventional methods.

The intermediate compounds of general formula (II) may be prepared from a compound of formula (IV):

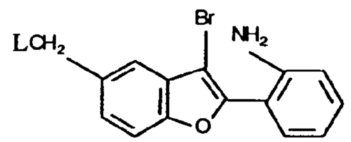

(IV)

(wherein L is a leaving group, for example, a halogen atom such as chlorine, bromine or iodine, or an alkyl- or arylsulphonyloxy group such as methanesulphonyloxy or p-toluenesulphonyloxy) with an imidazole of formula (V)

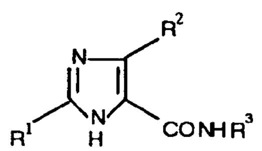

(V)

(wherein $R^1$, $R^2$ and $R^3$ are as defined in general formula (I)) followed by the removal of any protecting groups where present.

The reaction is preferably effected under basic conditions, for example, in the presence of sodium hydride, potassium carbonate or sodium methoxide. The reaction is conveniently effected in a solvent such as acetonitrile or an ether e.g. tetrahydrofuran or dioxan, a ketone e.g. butanone or methyl isobutyl ketone, or a substituted amide e.g. dimethylformamide, at a temperature between 0°C and the reflux temperature of the solvent.

It will be appreciated that the amine group in formula (IV) may be suitably protected, for example, using a carbamate group, such as t-butyl carbamate (BOC), readily cleaved by acid hydrolysis, or benzyl carbamate (CBZ), cleaved by catalytic hydrogenation.

The intermediate compounds of general formula (II) may also be prepared from a (suitably protected) compound of formula (VI)

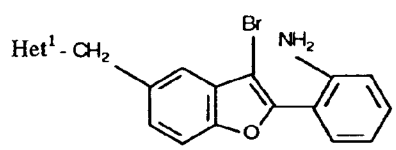

(VI)

(wherein $Het^1$ is as defined in formula (II) and in which X is a hydroxy group or $C_{1-6}$alkoxy group) according to the method of general process (B).

The intermediate compounds of formula (VI) may be prepared from a compound of formula (IV) and an imidazole of formula (VII)

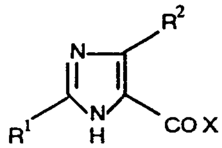

(VII)

(wherein $R^1$ and $R^2$ are as defined in general formula (I) and X is a $C_{1-6}$alkoxy group) according to the alkylation

conditions described herein above.

Where the group X represents a $C_{1-6}$alkoxy group, it may be conveniently hydrolysed to the corresponding acid using aqueous base, for example, aqueous potassium hydroxide in a suitable solvent, for example, aqueous ethanol, conveniently at room temperature.

The intermediates of formula (VI) may also be prepared from a (suitably protected) compound of formula (IV) and an imidazole of formula (VII) (in which X represents a hydrogen atom) using the alkylation conditions described herein above, followed by oxidation to the acid (X = hydroxy) under standard conditions, for example, using sodium chlorite, in a solvent such as aqueous tetrahydrofuran in the presence of an acid scavenger such as 2-methylbut-2-ene and a suitable buffer e.g. sodium dihydrogenorthophosphate.

The intermediate compound of formula (IV) may be prepared from a compound of formula (VIII)

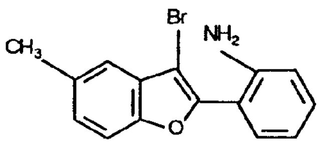
(VIII)

using any suitable reagent well known in the art for converting the methyl on the 6-membered ring into the group $-CH_2L$ (wherein L is as defined above). Thus, for example, when L is a halogen atom, a compound of formula (VIII) can be converted into a compound of general formula (IV) using N-chloro amides, tert-butyl hypochlorite or N-bromosuccinimide. Halogenation of the side chain may be catalysed by light, thus the reaction can be illuminated with a suitable artificial light source, and preferably in the presence of a free radical initiator such as azobisisobutyronitrile (AIBN) or benzoyl peroxide.

Compounds of formula (VIII) may be prepared by halogenation of the corresponding des-bromo compound of formula (IX)

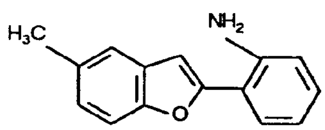
(IX)

using for example, bromine, in a suitable solvent such as a halogenated hydrocarbon, e.g. carbon tetrachloride.

Compounds of formula (IX) may be prepared by reaction of a compound of formula (X)

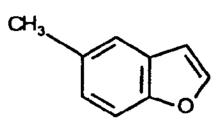
(X)

with a (protected) compound of formula (XI)

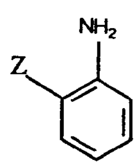
(XI)

(wherein Z represents a bromine or iodine atom or the group $-OSO_2CF_3$) followed, where necessary, by deprotection of the amino group.

The compound of formula (X) is first treated with an alkyl lithium compound such as n-butyl lithium at a reduced temperature, for example, between -100°C and 0°C in a solvent such as an ether (e.g. tetrahydrofuran). The mixture is then treated with a trialkylborate compound such as triisopropylborate and the temperature conveniently brought up to room temperature. Subsequently, water may be added and the mixture treated with a mineral acid such as sulphuric acid thus producing a compound of formula (Xa)

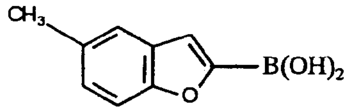

(Xa)

The intermediate compound of formula (Xa) is then reacted with a compound of formula (XI) in the presence of a palladium (0) compound such as tetrakis(triphenylphosphine) palladium (0) in a solvent such as an ether (e.g. dimethoxyethane), and in the presence of a base such as sodium carbonate or thallium hydroxide. The reaction is conveniently effected at an elevated temperature, such as the reflux temperature of the solvent.

Compounds of formula (VIII) may also be prepared by an intramolecular cyclisation reaction of a compound of formula (XII)

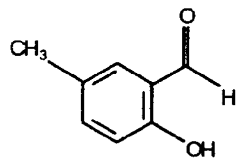

(XII)

with a suitably substituted benzene of formula (XIII)

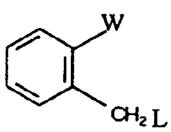

(XIII)

(wherein L is as previously defined and W is a group convertible to $NH_2$ such as $NO_2$ or a protected amine such as NHBOC (where BOC is a tert-butoxycarbonyl protecting group) followed by bromination of the 3-position on the benzofuran ring as described above.

The reaction is preferably effected in the presence of a base such as sodium hydride or potassium carbonate. The cyclisation is a two step reaction which requires one equivalent of base per step. It will be appreciated however that the reaction can be effected in the presence of two equivalents of base to avoid the need to isolate the intermediate. The reaction is conveniently effected in a solvent such as an ether e.g tetrahydrofuran, an alcohol e.g ethanol or a substituted amide e.g dimethylformamide, at a temperature between room temperature and the reflux temperature of the solvent.

The compounds of formula (II) may be prepared in an alternative manner by a Curtius rearrangement of a compound of formula (XIV)

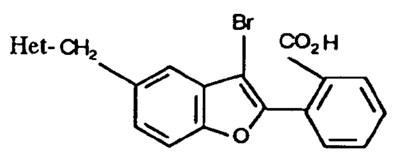

(XIV)

using, for example, diphenylphosphorylazide in the presence of a base such as triethylamine and in a solvent such as an alcohol (e.g. tert-butanol) to form a carbamate followed by deprotection of the amine in a conventional manner, for example, by acid hydrolysis using hydrochloric acid in a solvent such as ethanol.

The compounds of formula (XIV) may be prepared by methods analogous to those described herein commencing from a benzoic acid starting material rather than an aniline starting material.

The imidazoles of formulae (V) and (VII) may be prepared as described in European Specification No. 0253310A and in US Patent No. 4355040 or by methods analogous to those described therein. The content of these references is hereby incorporated by reference.

Intermediates of formulae (X), (XI), (XII) and (XIII) are either known compounds or may be prepared by methods analogous to those used for the preparation of the known compounds.

The following examples illustrate the invention. Temperatures are in °C. "Dried" refers to drying using magnesium sulphate. Thin layer chromatography (t.l.c.) was carried out on silica and column chromatography was carried out on silica (Merck 9385 unless otherwise stated), using one of the following solvent systems : A -

ether:hexane, B - ether: dichloromethane, C - dichloromethane:ethanol:conc. aqueous ammonia, D- dichloromethane:ethyl acetate, or E-dichloromethane:ether:acetic acid. The following abbreviations are used : THF - tetrahydrofuran; DME - dimethoxyethane; AIBN - azobisisobutyronitrile; DMF - dimethylformamide; TMEDA - tetramethylethylenediamine; NBS - N-bromosuccinimide; DMAP- 4-dimethylaminopyridine; DEAD - diethyl azodicarboxylate.

Intermediate 1

5-Methylbenzofuran-2-boronic acid

n-Butyl lithium (35.16ml) was added dropwise to a stirred solution of TMEDA (9.58ml) and 5-methylbenzofuran (8.22g) in ether (250ml) maintaining. the temperature below - 60°C throughout. The solution was warmed to about -10°C over 45 minutes and stirred at this temperature-for 30 minutes. A precipitate formed on warming. The suspension was cooled and triisopropylborate (43ml) was added, maintaining the temperature below - 60°C. The solution was warmed gradually to room temperature before quenching with 2N HCl (70ml). The mixture was extracted with ether (3x50ml) and the combined organic extracts washed with 2N HCl (4x30ml), water (2x30ml) and dried before evaporation to give the title compound as an orange solid (12.75g).
t.l.c. System A (1:1), Rf 0.3.

Intermediate 2

Methyl 2-(5-methyl-2-benzofuranyl)benzoate

A solution of methyl 2-bromobenzoate (11.70g), Intermediate 1 (12.75g) and tetrakistriphenylphosphine palladium (0) (0.5g) in DME (300ml) and 2N $Na_2CO_3$ (60ml) was heated to reflux with vigorous stirring under nitrogen. After 1.5h a further 500mg of catalyst was added and stirring at reflux under nitrogen continued. After about 5h the reaction was cooled to room temperature and diluted with ether (300ml). The organic layer was separated and washed with water (3x100ml) and dried. Filtration and evaporation gave a yellow oily suspension (19.27g) which was purified by chromatography eluting with System A (1:9) to give a yellow oil (11.06g). This was further purified by Kugelrohr distillation to give the title compound (4.31g). t.l.c. System A (1:9), Rf 0.5.

Intermediate 3

Methyl 2-(3-bromo-5-methyl-2-benzofuranyl)benzoate

A solution of Intermediate 2 (0.25g) in carbon tetrachloride (5ml) was cooled to -20°C and treated dropwise with 1M bromine in carbon tetrachloride (0.7ml). Stirring at -20°C was then continued for 1h before gradual warming to room temperature. Stirring at room temperature was continued overnight. Cyclohexene (0.1ml) was added dropwise and the solvents were evaporated in vacuo to give the title compound as an orange oil (0.26g).
t.l.c. System A (1:9), Rf 0.45.

Intermediate 4

2-(3-Bromo-5-methyl-2-benzofuranyl)benzoic acid

A solution of Intermediate 3 (2.20g) in methanol (20ml) was treated with sodium hydroxide (2N;3ml). The solution was heated to reflux and heating was continued for 3h. The solvent was removed in vacuo and the residue diluted with water. The basic aqueous phase was washed with ether (3x30ml) before acidification to pH~2 using 2N HCl. A white suspension formed. This was extracted with ether (4x20ml) and the combined organic extracts dried, filtered and evaporated to give the title compound as a pale yellow solid (1.93g).
T.l.c. ether, Rf 0.7

Intermediate 5

1,1-Dimethylethyl[2-(3-bromo-5-methyl-2-benzofuranyl) phenyl]carbamate

A solution of Intermediate 4 (1g) in dry dioxan (25ml) was treated with diphenylphosphorylazide (0.65ml), triethylamine (0.42ml) and tert-butanol (0.5ml) before heating to reflux under nitrogen. After 6h the reaction was

cooled and solvent evaporated to give an orange oil. Purification by column chromatography, eluting with System A (1:10) afforded the title compound as a cream solid (0.67g).
T.l.c. System A (1:1), Rf 0.8

Intermediate 6

1,1-Dimethylethyl [2-[3-bromo-5-(bromomethyl)-2-benzofuranyl] phenyl]carbamate

A solution of Intermediate 5 (4.29g), NBS (2.09g) and benzoyl peroxide (30mg) in dry carbon tetrachloride (100ml) was heated at reflux whilst being irradiated with a 200W lamp for 1.5 hours. The mixture was filtered, and the filtrate was washed with water (2x 100ml). The organic solution was dried, filtered and evaporated to give the title compound (5g).
t.l.c. System A (1:1) Rf = 0.73

Intermediate 7

2-Ethyl-1H-imidazole-5-methanol

Dihydroxyacetone (65g) was added slowly to liquid ammonia (250-300ml), followed by ethyl propanimidate hydrochloride (65g). The suspension was transferred to an autoclave and heated at 90°C for 16h. The ammonia was allowed to evaporate and then the reaction mixture concentrated in vacuo. The residue was dissolved in methanol, washed with charcoal, and then purified by chromatography, eluting with a gradient of System C (300:8:1 to 50:8:1). Trituration with acetone gave the title compound (29.83g) as a white solid.
T.l.c. System C (50:8:1) Rf 0.21

Intermediate 8

4-Chloro-2-ethyl-1H-imidazole-5-methanol

A solution of Intermediate 7 (19.66g) in 2-methoxyethanol (175ml) and 1,4-dioxan (175ml) was stirred with N-chlorosuccinimide (21.23g) in the dark at room temperature for 18h. The solvent was removed in vacuo and the residue partitioned between water, brine and ethyl acetate. The organic extracts were combined, backwashed with water and brine, dried and concentrated in vacuo to a yellow solid. This was triturated twice with dichloromethane to give the title compound as a white solid (11.49g).
T.l.c. System C (50:8:1) Rf 0.55

Intermediate 9

4-Chloro-2-ethyl-1H-imidazole-5-carboxaldehyde

Intermediate 8 (11,35g) was treated with magnesium dioxide (30.59g) in dichloromethane (240ml) and the mixture heated at reflux for 3.5h. The warm mixture was filtered and the filtrate concentrated in vacuo to a yellow solid. Trituration with petroleum ether followed by washing with ether gave the title compound as a white solid (6.41g)
T.l.c. System C (50:8:1) Rf 0.73

Intermediate 10

2-Ethyl-4-methyl-1H-imidazole-5-methanol

Concentrated HCl (75ml) was added to a solution of 2-ethyl-4-methyl-1H-imidazole (21g) and aqueous formaldehyde (37% 14ml) in water (100ml) and the mixture heated under reflux for 48h. The cooled mixture was basified to pH 10 with sodium hydroxide (5N) and extracted with chloroform /isopropanol (4:1) (3x100ml). The combined organic extracts were washed with brine (1x200ml) and dried. The solution was filtered and evaporated to give a pale yellow gum (18g) which was purified by column chromatography eluting with chloroform/methanol/conc. aqueous ammonia (90:10:1) the title compound as a pale yellow foam (12.6g)
T.l.c. chloroform/methanol/conc. aqueous ammonia (90:10:2) Rf 0.3

Intermediate 11

2-Ethyl-4-methyl-1H-imidazole-5-carboxaldehyde

Activated manganese dioxide (20g) was added to a suspension of Intermediate 10 (7.0g) in dichloromethane/1,4-dioxan (2:1) (200ml) and the mixture heated under reflux for 3h. The cooled mixture was filtered and the filtrate evaporated. Purification by column chromatography eluting with ether gave the title compound as a colourless crystalline solid (4.0g)
T.l.c. ether Rf 0.2

Intermediate 12

1-[[3-Bromo-2-[[(1,1-dimethylethoxy)carbonyl]amino]phenyl]-5-benzofuranyl]methyl]-4-chloro-2-ethyl-1H-imidazole-5-carboxaldehyde

A mixture of Intermediate 6 (9.5g), Intermediate 9 (2.95g) and potassium carbonate (3.1g) in DMF (30ml) was stirred at room temperature under a nitrogen atmosphere for 18h. The suspension was diluted with water (50ml) and extracted with diethyl ether (3x150ml). The combined extracts were dried and concentrated in vacuo to an oil which was purified by flash chromatography eluting with System A (3:7) to give the title compound (4.55g) as a white solid, m.p. 65-66°.

Similarly prepared were:

Intermediate 13

1-[[Bromo-2-[2-[[(1,1-dimethylethoxy)carbonyl]amino]phenyl]-5-benzofuranyl]methyl]-4-chloro-2-propyl-1H-imidazole-5-carboxaldehyde

m.p. 75-76°C
From Intermediate 6 and 4-chloro-2-propyl-1H-imidazole-5-carboxaldehyde.

Intermediate 14

1-[[3-Bromo-2-[2-[[(1,1-dimethylethoxy)carbonyl]amino]phenyl]-5-benzofuranyl]methyl]-2-ethyl-4-methyl-1H-imidazole-5-carboxaldehyde

n.m.r. (250MHz, $CDCl_3$) $\delta$ 1.34 (3H,t), 1.48 (9H,s), 2.55 (3H,s), 2.73 (2H,q), 5.69 (2H,s), 7.07-7.2 (3H,m), 7.07-7.2 (2H,m), 7.63 (1H,dd), 8.18 (1H, brd), 9.8 (1H,s)
From Intermediate 6 and Intermediate 11.

Intermediate 15

Ethyl 1-[[3-bromo-[2-[[(1,1-dimethylethoxy)carbonyl]amino] phenyl]-5-benzofuranyl]methyl]-2,4-diethyl-1H-imidazole-5-carboxylate

T.1.c. ether Rf = 0.48
From Intermediate 6 and ethyl 2,4-diethyl-1H-imidazole-5-carboxylate.

Intermediate 16

1,1-Dimethylethyl 2-[[3-bromo-5-[(5-formyl-4-methyl-2-propyl-1H-imidazol-1-yl)methyl]-2-benzofuranyl]phenyl]carbamate

T.1.c. ether:acetic acid (10:1) Rf = 0.45
From Intermediate 6 and 4-methyl-2-propyl-1H-imidazole-5-carboxaldehyde.

Intermediate 17

1-[[3-Bromo-2-[[(1,1-dimethylethoxy)carbonyl]amino]phenyl]-5-benzofuranyl]methyl]-4-chloro-2-ethyl-1H-imidazole-5-carboxylic acid

A solution of Intermediate 12 (4.25g) and 2-methyl-2-butene (2M in THF; 45.6ml) in dry THF (125ml) and t-butanol (29ml), under nitrogen atmosphere, was treated with a solution of sodium chlorite (80%; 6.87g) and sodium dihydrogen phosphate dihydrate (6.87g) in water (100ml). The mixture was stirred at room temperature for 20h. The layers were separated and the aqueous layer was extracted with diethyl ether (3x100ml). The combined extracts were washed with water (200ml), dried and concentrated in vacuo to give a yellow foam (4.8g). This material was dissolved in 2M sodium hydroxide solution (50ml) and then extracted once with diethyl ether (50ml). The basic layer was acidified to pH3 using 2M HCl. The resultant solid was collected by filtration to give the title compound (3.8g) as a white solid.
T.l.c.System E (10:2.5:0.5) Rf=0.78

Similarly prepared were:

Intermediate 18

1-[[3-Bromo-2-[[(1,1-dimethylethoxy)carbonyl]amino]phenyl]-5-benzofuranyl]methyl]-4-chloro-2-propyl-1H-imidazole-5-carboxylic acid

T.1.c. System E (10:2.5:0.5) Rf = 0.78
From Intermediate 13.

Intermediate 19

1-[[3-Bromo-2-[[(1,1-dimethylethoxy)carbonyl]amino]phenyl]-5-benzofuranyl]methyl]-2-ethyl-4-methyl-1H-imidazole-5-carboxylic acid

n.m.r. (250MHz, DMSO $d_6$) δ 1.14 (3H,t), 2.42 (3H,s), 2.62 (2H,q), 5.73 (2H,s), 6.09 (1H,dd), 7.18 (1H,brs), 7.27 (1H,dt), 7.44 - 7.64 (4H,m), 8.98 (1H,br.s).
From Intermediate 14.

Intermediate 20

1-[[3-Bromo-2-[2-[[(1,1-dimethylethoxy)carbonyl]amino]phenyl]-5-benzofuranyl]methyl]-4-methyl-2-propyl-1H-imidazole-5-carboxylic acid

T.1.c. dichloromethane:methanol (10:1) Rf = 0.3
From Intermediate 16.

Intermediate 21

Ethyl 1-[[3-bromo-2-[2-[[(1,1-dimethylethoxy)carbonyl]amino]phenyl]-5-benzofuranyl]methyl]-4-chloro-2-ethyl-1H-imidazole-5-carboxylate

A solution of DEAD (0.62ml) in dry THF (10ml) was added, dropwise, at room temperature under nitrogen atmosphere to a solution of Intermediate 17 (1.5g) and triphenylphosphine (1.023g) in ethanol (0.45ml) and dry THF (65ml). The resulting solution was stirred at room temperature for 20h, and then concentrated in vacuo. The residue was purified by flash chromatography eluting with System A (3:7) to give the title compound (1.2g) as a white foam, m.p. 73-4°.
Similarly prepared were:-

Intermediate 22

Ethyl 1-[[3-bromo-2-[2-[[(1,1-dimethylethoxy)carbonyl]amino]phenyl]-5-benzofuranyl]methyl]-4-chloro-2-propyl-1H-imidazole- 5-carboxylate

m.p. 68-70°C
From Intermediate 18.

Intermediate 23

Ethyl 1-[[3-bromo-2-[2-[[(1,1-dimethylethoxy)carbonyl]amino]phenyl]-5-benzofuranyl]methyl]-2-ethyl-4-methyl-1H-imidazole-5-carboxylate

n.m.r. (250 MHz, $CDCl_3$) δ 1.31 (3H,t), 1.32 (3H,t), 1.48 (9H,s), 2.55 (3H,s), 2.7 (2H,q), 4.28 (2H,q), 5.68 (2H,s), 7.04 (1H,dd), 7.1-7.3 (3H,m), 7.4-7.5 (2H,m), 7.63 (1H,dd), 8.19 (1H,d).
From Intermediate 19.

Intermediate 24

Ethyl 1-[[3-bromo-2-(2-aminophenyl)-5-benzofuranyl]methyl]-4-chloro-2-ethyl-1H-imidazole-5-carboxylate

A solution of Intermediate 21 (1.2g) in dry dichloromethane (30ml), under nitrogen atmosphere, was cooled to 0°C and trifluoroacetic acid (6ml) was added. The solution was stirred at room temperature for 3h and was then concentrated in vacuo. Methanolic ammonia was added to the residue and the mixture was concentrated in vacuo. Purification of the residue by flash chromatography eluting with ether gave the title compound (0.8g) as a white foam, m.p. 53-56°.
Similarly prepared were :-

Intermediate 25

Ethyl 1-[[3-bromo-2-(2-aminophenyl)-5-benzofuranyl]methyl]-4-chloro-2-propyl-1H-imidazole- 5-carboxylate

m.p. 45-46°C
From Intermediate 22.

Intermediate 26

Ethyl 1-[[3-bromo-2-(2-aminophenyl)-5-benzofuranyl]methyl]-2-ethyl-4-methyl-1H-imidazole-5-carboxylate

T.1.c. System C (100:8:1) Rf = 0.5
From Intermediate 23.

Intermediate 27

Ethyl 1-[[3-bromo-[2-(2-aminophenyl)-5-benzofuranyl]methyl]-2,4-diethyl-1H-imidazole- 5-carboxylate

m.p. 54-55°C
From Intermediate 15.

Intermediate 28

Ethyl 1-[[3-bromo-[2-[[(trifluoromethyl)sulphonyl]amino]phenyl]-5-benzofuranyl]methyl]-4-chloro-2-ethyl-1H-imidazole-5-carboxylate

A solution of Intermediate 24 (0.2g) and dry triethylamine (0.055ml) in dry dichloromethane (9.5ml) was cooled to -95°C, under nitrogen, and a solution of trifluoromethanesulphonic anhydride (0.1ml) in dry dichloromethane (0.5ml) was added. The resulting solution was stirred at -95°C to -75°C for 2h. Water (2ml) was added at -70°C and then the mixture was warmed to room temperature. The organic solution was dried and concentrated in

vacuo. Purification by column chromatography eluting with ether gave a foam (0.23g), a portion of which was recrystallised from methyl acetate/hexane to give the title compound as a white solid (0.138g) m.p. 89-90°C. Similarly prepared were:-

Intermediate 29

Ethyl 1-[[3-bromo-2-[2-[[(trifluoromethyl)sulphonyl]amino]phenyl]-5-benzofuranyl]methyl]-4-chloro-2-propyl-1H-imidazole-5-carboxylate

m.p. 77-78°C
From Intermediate 25.

Intermediate 30

Ethyl 1-[[3-bromo-2-[2-[[(trifluoromethyl)sulphonyl]amino]phenyl]-5-benzofuranyl]methyl]-2-ethyl-4-methyl-1H-imidazole-5-carboxylate

m.p. 105-107°C
From Intermediate 26.

Intermediate 31

Ethyl 1-[[3-bromo-2-[2-[[(trifluoromethyl)sulphonyl]amino]phenyl]-5-benzofuranyl]methyl]-2,4-diethyl-1H-imidazole-5-carboxylate

m.p.99-101°C
From Intermediate 27.

Intermediate 32

1-[[3-Bromo-2-[2-[[(trifluoromethyl)sulphonyl]amino]phenyl]-5-benzofuranyl]methyl]-4-chloro-2-ethyl-1H-imidazole-5-carboxylic acid

A solution of Intermediate 28 (0.4g) in methanol (20ml) and 2M NaOH (4ml) was heated at reflux for 1.5h. The solution was concentrated in vacuo. It was then diluted with water (10ml) and extracted with diethyl ether (10ml). The aqueous layer was acidified to pH5 with 2N HCl and extracted with ethyl acetate (3x20ml). The combined organic extracts were dried and concentrated in vacuo to give the title compound (0.35g) as a pale beige solid.
T.l.c. dichloromethane/methanol (10:0.5) Rf=0.43
Similarly prepared were:-

Intermediate 33

1-[[3-Bromo-2-[2-[[(trifluoromethyl)sulphonyl]amino]phenyl]-5-benzofuranyl]methyl]-4-chloro-2-propyl-1H-imidazole-5-carboxylic acid

m.p. 174-175°C (dec)
From Intermediate 29.

Intermediate 34

1-[[3-Bromo-2-[2-[[(trifluoromethyl)sulphonyl]amino]phenyl]-5-benzofuranyl]methyl]-2-ethyl-4-methyl-1H-imidazole-5-carboxylic acid

m.p. 153-155°C
From Intermediate 30.

Intermediate 35

1-[[3-Bromo-2-[2-[[(trifluoromethyl)sulphonyl]amino]phenyl]-5-benzofuranyl]methyl]-2,4-diethyl-1H-imidazole-5-carboxylic acid

T.1.c dichloromethane:methanol (10:1) Rf = 0.14
From Intermediate 31.

Intermediate 36

Ethyl 1-[[3-bromo-2-[2-[[(1,1-dimethylethoxy)carbonyl]amino]phenyl]-5-benzofuranyl]methyl]-4-chloro-2-ethyl-N-methyl-1H-imidazole-5-carboxamide

Carbonyldiimidazole (0.507g) was added to a solution of Intermediate 17 (0.6g) in dry THF (30ml), under nitrogen atmosphere, and the mixture was stirred at room temperature for 4h. Methylamine 40% aqueous solution (1.3ml) was added and the resulting solution was stirred at room temperature for 2h. The solution was concentrated in vacuo and the residue was purified by flash chromatography eluting with diethyl ether to give the title compound (0.4g) as a white solid, m.p. 95-6 °.
Similarly prepared were:-

Intermediate 37

1-[[3-Bromo-2-[2-[[(1,1-dimethylethoxy)carbonyl]amino]phenyl]-5-benzofuranyl]methyl]-2-ethyl-N,4-dimethyl-N-methyl-1H-imidazole-5-carboxamide

n.m.r. (250 MHz, $CDCl_3$) δ 1.34 (3H,t), 1.48 (9H,s), 2.53 (3H,s), 2.76 (2H,q), 2.96 (3H,d), 5.63 (2H,s), 6.1-6.4 (1H, vbr.s), 7.08-7.2 (3H,m), 7.28 (1H,dd), 7.42-7.52 (2H,m), 7.62 (1H,dd), 8.15 (1H, br.d)
From Intermediate 19 and methylamine.

Intermediate 38

1-[[3-Bromo-2-[2-[[(1,1-dimethylethoxy)carbonyl]amino]phenyl]-5-benzofuranyl]methyl]-4-methyl-2-propyl-1H-imidazole-5-carboxamide

T.1.c. dichloromethane:methanol (10:1) Rf = 0.4
From Intermediate 20 and conc. aqueous ammonia.

Intermediate 39

1-[[3-Bromo-2-2[2-[[(1,1-dimethylethoxy)carbonyl]amino]phenyl]-5-benzofuranyl]methyl]-N-ethyl-4-methyl-2-propyl-1H-imidazole-5-carboxamide

T.1.c. dichloromethane:methanol (10:1) Rf= 0.57
From Intermediate 20 and ethylamine.

Intermediate 40

1-[[3-Bromo-2-(aminophenyl)-5-benzofuranyl]methyl]-4-chloro-2-ethyl-N-methyl-1H-imidazole-5-carboxamide

A solution of Intermediate 36 (0.4g) in dry dichloromethane (20ml), under nitrogen atmosphere, was cooled to 0°C and trifluoroacetic acid (2ml) was added. The resulting solution was stirred at room temperature for 3h. Water (10ml) was added and then aqueous ammonia (10ml). The layers were separated. The organic phase was dried and concentrated in vacuo. The residue was purified by flash chromatography eluting with ether to give the title compound (0.3g) as a white foam, m.p. 68-70°.
Similarly prepared were :-

Intermediate 41

1-[[3-Bromo-2-(2-aminophenyl)-5-benzofuranyl]methyl]-2-ethyl-N,4-dimethyl-1H-imidazole-5-carboxamide

T.1.c. System C (100:8:1) Rf = 0.4
From Intermediate 37.

Intermediate 42

1-[[3-Bromo-2-(2-aminophenyl)-5-benzofuranyl]methyl]-N-ethyl-4-methyl-2-propyl-1H-imidazole-5-carboxamide

T.1.c. dichloromethane:methanol (10:1) Rf= 0.54
From Intermediate 39.

Intermediate 43

1-[[3-Bromo-2-(2-aminophenyl)-5-benzofuranyl]methyl]-4-methyl-2-propyl-1H-imidazole-5-carboxamide

T.l.c. dichloromethane:methanol (10:1) Rf = 0.25
From Intermediate 38.

Intermediate 44

Ethyl 4-methyl-2-propryl-1H-imdiazole-5-carboxylate

Ethyl 2-amino-3-oxobutanoate hydrochloride (10g) was added to a stirring solution of ethyl butaneimidate (83.5g) in ethanol (800ml, freshly distilled from magnesuim ethoxide) and triethylamine (85ml) and the resultant yellow mixture stirred at room temperature for 48h. The solvent was removed in vacuo and the residue diluted with water (500ml) and extracted into ethyl acetate (3x200ml). The combined organic extracts were washed with water (2x100ml), dried and concentrated in vacuo. The resultant residue was purified by trituration with ether (5x50ml) and the residual buff solid dried to afford the title compound (4.05g)
T.l.c ether Rf = 0.31

Intermediate 45

Ethyl 1-[[3-bromo-2-[2-[[(1,1-dimethylethoxy)carbonyl]amino]phenyl]-5-benzofuranyl]methyl]-4-methyl-2-propyl-1H-imidazole- 5-carboxylate

A solution of Intermediate 44 (0.51g) and sodium hydride (0.11g; 60% dispersion in oil) in dry DMF (6ml) was stirred at ambient temperature for 2 hours. The mixture was cooled to 0 to 5° and Intermediate 6 (1.25g) was added. The reaction mixture was stirred at 0° rising to ambient temperature for 20 hours. The solvent was evaporated in vacuo and the residue was dissolved in ether (100ml). The ethereal solution was washed with water (100ml) aqueous sodium bicarbonate solution (8%;100ml), aqueous lithium chloride solution (10%;50ml), dried and evaporated. The residue was purified by column chromatography to give the title compound as an off-white foam (0.2g).
T.1.c. System B (2:25) Rf = 0.65

Intermediate 46

Ethyl 1-[[2-(2-aminophenyl)-3-bromo-5-benzofuranyl]methyl]-4-methyl-2-propyl-1H-imidazole-5-carboxylate

A solution of Intermediate 45 (0.19g) in a mixture of dichloromethane (3ml) and trifluoroacetic acid (1ml) was stirred at 0° to ambient temperature for 4 hours. The solvent was removed in vacuo and ethanol (5ml) and ammonia (1ml) were added and the solution was re-evaporated. The residue was purified by column chromatography eluting with dichloromethane/methanol (20:1) to give the title compound (0.136g) as a yellow foam, m.p. 135-6°C.

Intermediate 47

Ethyl 1-[[3-bromo-2-[2-[[(trifluoromethyl)sulphonyl]amino]phenyl]-5-benzofuranyl]methyl]-4-methyl-2-propyl-1H-imidazole- 5 carboxylate

A solution of Intermediate 46 (0.133g) and dry triethylamine (33mg) in dry dichloromethane (5ml) at -85° was treated with a solution of trifluoromethane sulphonyl anhydride in dry dichloromethane (0.33ml; 1M). The mixture was stirred at -85° to -75° for 3h and water (5ml) was added. The product was extracted with dichloromethane (20ml). The organic extract was washed with hydrochloric acid (2M; 20ml), dried, filtered and evaporated in vacuo. The residue was purified by column chromatography eluting with dichloromethane/methanol (50:1) to give the title compound as a white solid (0.135g), m.p. 123-5°.

Intermediate 48

1-[[3-Bromo-2-[2-[[(trifluoromethyl)sulphonyl]amino]phenyl]-5-benzofuranyl]methyl]-4-methyl-2-propyl-1H-imidazoles-5-carboxylic acid

A solution of Intermediate 47 (0.13g) in a mixture of methanol (6ml) and aqueous sodium hydroxide solution (2M; 2ml) was heated at reflux for 3h. Hydrochloric acid (2M; 2ml) was added to the cooled mixture and the resulting precipitate was collected by filtration. The solid was crystallized from methanol and water to give the title compound as a white solid, (0.06g) m.p. 168-170°.

Example 1

1-[[3-Bromo-2-[2-[[(trifluoromethyl)sulphonyl]amino]phenyl]-5-benzofuranyl]methyl]-4-chloro-2-ethyl-1H-imidazole-5-carboxamide

Carbonyldiimidazole (0.12g) was added to a solution of Intermediate 32 (0.15g) in dry THF (5ml) under a nitrogen atmosphere. The resulting solution was stirred at room temperature for 3h. Ammonia (35% aqueous solution; 0.2ml) was added and the resulting solution was stirred at room temperature for 2h. The solution was concentrated in vacuo, then diluted with ethyl acetate (20ml) and washed with water (2x15ml). The organic layer was dried and concentrated in vacuo to a solid which was crystallized from methyl acetate/n-hexane to give the title compound (0.14g) as a white solid, m.p. 68-70C.
T.l.c. System E (10:2.5:0.5) Rf=0.43
Similarly prepared were:-

Example 2

1-[[3-Bromo-2-[2-[[(trifluoromethyl)sulphonyl]amino]phenyl]-5-benzofuranyl]methyl]-4-chloro-N-methyl-2-propyl-1H-imidazole-5-carboxamide

m.p. 78-80°C
T.1.c. chloroform/methanol (20:2.5) Rf = 0.61
From Intermediate 33 and methylamine.

Example 3

1-[[3-Bromo-2-[2-[[(trifluoromethyl)sulphonyl]amino]phenyl]-5-benzofuranyl]methyl]-4-chloro-2-propyl-1H-imidazole-5-carboxamide

m.p. 198-200°C

| | |
|---|---|
| Analysis Found: | C,44.6; H.3.0; N, 8.95 |
| $C_{23}H_{19}BrClF_3N_4O_4S$ requires | C,44.6; H.3.1, N, 9.0% |

From Intermediate 33.

Example 4

1-[[3-Bromo-2-[2-[[(trifluoromethyl)sulphonyl]amino]phenyl]-5-benzofuranyl]methyl]-2-ethyl-4-methyl-1H-imidazole-5-carboxamide

m.p. 118-124°C.
T.1.c. dichloromethane:methanol:acetic acid (20:4:1) Rf =0.45
From Intermediate 34.

Example 5

1-[[3-Bromo-2-[2-[[(trifluoromethyl)sulphonyl]amino]phenyl]-5-benzofuranyl]methyl]-N,4-dimethyl-2-propyl-1H-imidazole-5-carboxamide

m.p. 164-170°C

| | |
|---|---|
| Analysis Found: | C, 49.1; H, 4.1; N, 8.8 |
| $C_{25}H_{24}BrF_3N_4O_4S$ requires: | C, 48.9; H, 3.9; N, 9.1% |

From Intermediate 48 and methylamine.

Example 6

1-[[3-Bromo-2-[2-[[(trifluoromethyl)sulphonyl]amino]phenyl]-5-benzofuranyl]methyl]-2,4-diethyl-1H-imidazole-5-carboxamide

m.p. 146-151°C (decomp)
T.1.c. dichloromethane:methanol (15:1) Rf = 0.23
From Intermediate 35.

Example 7

1-[[3-Bromo-2-[2-[[(trifluoromethyl)sulphonyl]amino]phenyl]-5-benzofuranyl]methyl]-2,4-diethyl-N-methyl-1H-imidazole-5-carboxamide

m.p. 157-162°C (decomp)
T.1.c. dichloromethane:methanol (10:1) Rf = 0.48
From Intermediate 35 and methylamine.

Example 8

1-[[3-Bromo-2-[2-[[(trifluoromethyl)sulphonyl]amino]phenyl]-5-benzofuranyl]methyl]-N,2-diethyl-4-methyl-1H-imidazole-5-carboxamide

1,1-Carbonyldiimidazole (0.181g) was added to a stirred solution of Intermediate 34 (0.403g) in dry THF (25ml). After stirring for 4h at room temperature the solution was allowed to stand at room temperature overnight. 70% Aqueous ethylamine (15ml) was added and the mixture heated with stirring for 1.25h. After cooling, the solution was partitioned between ethyl acetate (30ml) and 10% brine (30ml). The separated aqueous phase was further extracted with ethyl acetate (3x20ml) and the combined organic extracts were dried, concentrated in vacuo and azeotroped with toluene (3x15ml) to afford an orange oil (0.7g). This oil was dissolved in ethyl acetate (40ml) and washed with 2N hydrochloric acid (1x30ml, 1x15ml), dried and concentrated in vacuo to afford an off-white foam (0.31g). Purification by chromatography eluting with a gradient of System C (150:8:1) → (50:8:1) afforded the title compound (0.25g) as a white foam, m.p. 110°-115° (softens), melts 140°.
N.m.r. - ($DMSOd_6$) δ 1.08 (t,3H), 1.25 (t,3H), 2.3 (s,3H), 3.0 (q,2H), 3.28 (m,2H), 5.6 (br s,2H), 6.8-7.6 (m,8H), 8.5 (m,1H).
Similarly prepared were:-

Example 9

1-[[3-Bromo-2-[2-[[(trifluoromethyl)sulphonyl]amino]phenyl]-5-benzofuranyl]methyl]-4-chloro-N,2-diethyl-1H-imidazole-5-carboxamide

m.p. 104-110°C
T.1.c. dichloromethane:methanol (10:1) Rf = 0.7
From Intermediate 32.

Example 10

1-[[3-Bromo-2-[2-[[(trifluoromethyl)sulphonyl]amino]phenyl]-5-benzofuranyl]methyl]-4-chloro-N-ethyl-2-propyl-1H-imidazole-5-carboxamide

m.p. 172°C

| | |
|---|---|
| Analysis Found | C, 46.7; H,3.7; N. 8.45 |
| $C_{25}H_{23}BrClF_3N_4O_4S$ requires | C, 46.35; H,3.6; N, 8.65% |

From Intermediate 33.

Example 11

1-[[3-Bromo-2-[2-[[(trifluoromethyl)sulphonyl]amino]phenyl]-5-benzofuranyl]methyl]-N,2,4-triethyl-1H-imidazole-5-carboxamide

T.1.c. dichloromethane:methanol (4:1) Rf = 0.7
n.m.r. (DMSO $d_6$) $\delta$ 1.08 (3H,t), 1.15 (3H,t), 1.22 (3H,t), 2.65 (2H,q), 2.85 (2H,q), 3.25 (2H,m), 5.52 (2H,s), 6.88 (1H,t), 7.15-7.55 (6H,m), 8.25 (1H,m).
From Intermediate 35.

Example 12

1-[[3-Bromo-2-[2-[[(trifluoromethyl)sulphonyl]amino]phenyl]-5-benzofuranyl]methyl]-4-chloro-2-ethyl-N-methyl-1H-imidazole-5-carboxamide

A solution of Intermediate 40 (0.28g) and dry triethylamine (0.08ml) in dry dichloromethane (9ml) was cooled to -95°C, under nitrogen atmosphere, and a solution of trifluoromethanesulphonic anhydride (0.116ml) in dry dichloromethane (1ml) was added. The mixture was stirred at -95° to -75° for 1h. Water (5ml) was added, the layers were separated and the organic phase was washed with 2N HCl (10ml). The organic solution was dried and concentrated in vacuo to give a foam which was purified by flash chromatography eluting with chloroform/methanol (30:1) to give the title compound (0.2g) as a white solid, m.p. 105-6° (dec.).
T.1.c. chloroform/methanol (30:1) Rf=0.47
Similarly prepared were:-

Example 13

1-[[3-Bromo-2-[2-[[(trifluoromethyl)sulphonyl]amino]phenyl]-5-benzofuranyl]methyl]-2-ethyl-4-methyl-N-methyl-1H-imidazole-5-carboxamide

m.p. 170-173°C

| | |
|---|---|
| Assay Found: | C,47.75; H,3.75; N,9.35; |
| $C_{29}H_{32}BrN_3O_5$ requires: | C,48.1; H,3.7; N, 8.9% |

From Intermediate 41.

Example 14

1-[[3-Bromo-2-[2-[[(trifluoromethyl)sulphonyl]amino]phenyl]-5-benzofuranyl]methyl]-N-ethyl-4-methyl-2-propyl-1H-imidazole-5-carboxamide

m.p. 158-164°C
T.1.c. (on alumina) dichloromethane:methanol:acetic acid (50:10:2)
Rf = 0.4
From Intermediate 42.

Example 15

1-[[3-Bromo-2-[2-[[(trifluoromethyl)sulphonyl]amino]phenyl]-5-benzofuranyl]methyl]-4-methyl-2-propyl-1H-imidazole-5-carboxamide

m.p. 250°C (dec.)
T.1.c. dichloromethane:methanol (10:1) Rf = 0.19
From Intermediate 43.

The compounds of the invention are tested in vitro for angiotensin II receptor antagonism. Aortic strips are obtained from male New Zealand white rabbits and prepared for recording isometric contractions in response to cumulative addition of angiotensin II. The potencies of test antagonists are assessed by measuring their abilities to displace the angiotensin II cumulative concentration response curve. The method used is that of Ackerly et al., Proc. Natl. Acad. Sci., 74(12), pp5725-28 (1977) with the exception that the final composition of the physiological salt solution is as given below in Table 1:

TABLE 1

| Ingredient | Amount (mM) |
|---|---|
| $Na^+$ | 143.4 |
| $K^+$ | 5.9 |
| $Mg^{2+}$ | 0.6 |
| $Ca^{2+}$ | 1.3 |
| $Cl^-$ | 124.5 |
| $HPO^{4-}$ | 1.2 |
| $SO_4^{2-}$ | 0.6 |
| $HCO_3^-$ | 25.0 |
| glucose | 11.1 |
| indomethacin | 0.005 |
| ascorbic acid | 0.1 |

The tissues are initially challenged with $K^+$ (80mM) and then washed at 0, 5, 10 and 15 minutes after the response to $K^+$ has reached a plateau. After a further 45 minutes an angiotensin II cumulative response curve is constructed (0.1nM to 0.1μM in 10-fold increments) and the tissues are washed as before. A second, third and fourth angiotensin II cumulative response curve (0.1nM to 0.1μM in 3-fold increments) is then constructed at hourly intervals (15 minutes washing after each curve followed by 45 minutes equilibration). The compounds of the invention (30μM) are tested for angiotensin II receptor antagonism by application 45 minutes before construction of the fourth angiotensin II curve. The third and fourth angiotensin II curves are expressed graphically and a concentration ratio (CR) is calculated by dividing the angiotensin II $EC_{50}$ value obtained in the presence of the test antagonist (i.e. fourth curve) by the angiotensin II $EC_{50}$ value obtained in the absence of the test antagonist (i.e. third curve).

The potency of the test antagonist is expressed as a pKb which is calculated from the equation :

$$pKb = -\log \left[ \frac{CR-1}{[antagonist]} \right]$$

which is a rearrangement of equation 4 described by Furchgott, in Handbook of Exp. Pharmacol., 33, p290 (1972) (eds. Blaschko and Muscholl).

If a compound supresses the maximum response to angiotensin II, a pKb is estimated using the double reciprocal plot technique for insurmountable antagonists, described by T.P. Kenakin, Pharmacol. Rev., 36(3), pp165-222 (esp. 203-204) (1984).

Compounds of the invention will desirably exhibit a pKb in the range between 5 and 12. Thus we have found that the compounds of the invention inhibit the action of the hormone angiotensin II and are therefore useful in the treatment of conditions in which it is desirable to inhibit angiotensin II activity. In particular, the compounds of the Examples are active in the above test.

There is thus provided as a further aspect of the invention a compound of general formula (I) or a physiologically acceptable salt or solvate thereof for use in the treatment of conditions associated with excessive or unregulated angiotensin II activity.

In a further or alternative aspect of the invention there is provided a compound of general formula (I) or a physiologically acceptable salt or solvate thereof for the manufacture of a therapeutic agent for the treatment of conditions associated with excessive or unregulated angiotensin II activity.

There is also provided in a further or alternative aspect of the invention a method for the treatment of conditions associated with excessive or unregulated angiotensin II activity in a mammal including man comprising administration of an effective amount to a mammal in need of such treatment a compound of general formula (I) or a physiologically acceptable salt or solvate thereof.

In addition, by virtue of their antagonistic activity at angiotensin II receptors, compounds of the present invention will be of value in the treatment of conditions associated with activation of the Renin-Angiotensin System.

There is thus provided a further aspect of the present invention a compound of general formula (I) or a physiologically acceptable salt, solvate or metabolically labile ester thereof for use in the treatment of a condition associated with activation of the Renin-Angiotensin system.

In a further or alternative aspect of the present invention there is provided a compound of general formula (I) or a physiologically acceptable salt, solvate or metabolically labile ester thereof for the manufacture of a therapeutic agent for the treatment of a condition associated with activation of the Renin-Angiotensin System.

There is also provided in a further or alternative aspect of the present inventions a method for the treatment of a condition associated with the activation of the Renin-Angiotensin System in a mammal including man comprising administration of an effective amount to a mammal in need of such treatment of a compound of general formula (I) or a physiologically acceptable salt, solvate or metabolically labile ester thereof.

Plasma concentrations of the compounds of the present invention following oral and intra-arterial administration to conscious normotensive dogs is measured in the following manner:

Spiked or unspiked plasma (0.5ml) is acidified using 1M HCl (0.1ml) and diluted to 1ml in aqueous TFA (0.05% v/v). These samples are centrifuged and the supernatant fractions (0.5ml) are applied onto $C_{18}$ Bond Elat cartridges pre-conditioned with methanol (3ml) and water (3ml). Samples are eluted using methanol, evaporated in vacuo and resuspended in hplc mobile phase (150-200$\mu$l) prior to centrifugation and analysis by hplc [5$\mu$, 15 x 0.46cm i.d. column; mobile phase = acetonitrile/water (containing 0.05% v/v TFA), 40-48% water; flow rate = 1ml/minute; Detection $\lambda$ = 299nm]. The concentration of each compound in the plasma sample is determined against calibration standards.

Compounds of the present invention are also analysed for their anti-hypertensive activity in vivo using renal-ligated hypertensive rats. Table 1 below shows the reduction in diastolic blood pressure after 7 hours following administration of test compounds (0.5mg/kg) to the rats, both orally (p.o.) and intra-arterially (i.a.):

TABLE 1

| Test Compound | Reduction in diastolic blood pressure after 7h. p.o. | i.a. |
|---|---|---|
| Example 1 | -55 | -49 |
| Example 2 | -60 | -43 |
| Example 12 | -31 | -42 |
| Example 13 | -54 | -39 |

The following.examples illustrate pharmaceutical formulations according to the invention. The term "active ingredient" is used herein to represent a compound of formula (I).

Pharmaceutical Example 1

Oral Tablet A

| | |
|---|---|
| Active Ingredient | 700mg |
| Sodium starch glycollate | 10mg |
| Microcrystalline cellulose | 50mg |
| Magnesium stearate | 4mg |

Sieve the active ingredient and microcrystalline cellulose through a 40 mesh screen and blend in a appropriate blender. Sieve the sodium starch glycollate and magnesium stearate through a 60 mesh screen, add to the powder blend and blend until homogeneous. Compress with appropriate punches in an automatic tablet press. The tablets may be coated with a thin polymer coat applied by the film coating techniques well known to those skilled in the art. Pigments may be incorporated in the film coat.

Pharmaceutical Example 2

Oral Tablet B

| | |
|---|---|
| Active Ingredient | 500mg |
| Lactose | 100mg |
| Maize Starch | 50mg |
| Polyvinyl pyrrolidone | 3mg |
| Sodium starch glycollate | 10mg |
| Magnesium stearate | 4mg |
| Tablet Weight | 667mg |

Sieve the active ingredient, lactose and maize starch through a 40 mesh screen and blend the powders in a suitable blender. Make an aqueous solution of the polyvinyl pyrrolidone (5 - 10% w/v). Add this solution to the blended powders and mix until granulated; pass the granulate through a 12 mesh screen and dry the granules in a suitable oven or fluid bed dryer. Sieve the remaining components through a 60 mesh screen and blend them with the dried granules. Compress, using appropriate punches, on an automatic tablet press.

The tablets may be coated with a thin polymer coat applied by film coating techniques well known to those skilled in art. Pigments may be incorporated in the film coat.

Pharmaceutical Example 3

| Inhalation Cartridge | |
|---|---|
| Active Ingredient | 1mg |
| Lactose | 24mg |

Blend active ingredient, particle size reduced to a very fine particle size (weight mean diameter ca. 5µm) with the lactose in a suitable powder blender and fill the powder blender into No. 3 hard gelatin capsules.

The contents of the cartridges may be administered using a powder inhaler.

Pharmaceutical Example 4

Injection Formulation

| | | % w/v |
|---|---|---|
| Active ingredient | | 1.00 |
| Water for injections B.P. | to | 100.00 |

Sodium chloride may be added to adjust the tonicity of the solution and the pH may be adjusted to that of maximum stability and/or to facilitate solution of the active ingredient using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

**Claims**

1. A compound of the general formula (I):

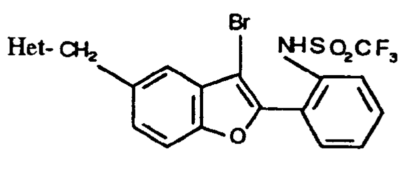

(I)

or a physiologically acceptable salt or solvate thereof wherein
Het represents an N-linked imidazolyl group of the formula

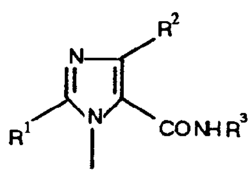

;

$R^1$ represents an ethyl or n-propyl group;
$R^2$ represents a chlorine atom or a methyl or ethyl group; and
$R^3$ represents a hydrogen atom or a methyl or ethyl group.

2. A compound as claimed in Claim 1 wherein $R^2$ represents a chlorine atom or a methyl group.

3. A compound as claimed in Claim 1 selected from

1-[[3-bromo-2-[2-[[(trifluoromethyl)sulphonyl]amino]phenyl]-5-benzofuranyl]methyl]-N,4-dimethyl-2-ethyl-1H-imidazole-5-carboxamide;
1-[[3-bromo-2-[2-[[(trifluoromethyl)sulphonyl]amino]phenyl]-5-benzofuranyl]methyl]-4-chloro-2-ethyl-1H-imidazole-5-carboxamide;
1-[[3-bromo-2-[2-[[(trifluoromethyl)sulphonyl]amino]phenyl]-5-benzofuranyl]methyl]-4-chloro-N-methyl-2-propyl-1H-imidazole-5-carboxamide;
1-[[3-bromo-2-[2-[[(trifluoromethyl)sulphonyl]amino]phenyl]-5-benzofuranyl]methyl]-4-chloro-N-ethyl-2-propyl-1H-imidazole-5-carboxamide;
1-[[3-bromo-2-[2-[[(trifluoromethyl)sulphonyl]amino]phenyl]-5-benzofuranyl]methyl]-4-chloro-2-ethyl-N-methyl-1H-imidazole-5-carboxamide;
1-[[3-bromo-2-[2-[[(trifluoromethyl)sulphonyl]amino]phenyl]-5-benzofuranyl]methyl]-2-ethyl-4-methyl-1H-imidazole-5-carboxamide;
1-[[3-bromo-2-[2-[[(trifluoromethyl)sulphonyl]amino]phenyl]-5-benzofuranyl]methyl]-N,4-dimethyl-2-propyl-1H-imidazole-5-carboxamide;
1-[[3-bromo-2-[2-[[(trifluoromethyl)sulphonyl]amino]phenyl]-5-benzofuranyl]methyl]-4-methyl-2-propyl-1H-imidazole-5-carboxamide;
or a physiologically acceptable salt or solvate thereof.

4. A compound as claimed in Claim 1 selected from
1-[[3-bromo-2-[2-[[(trifluoromethyl)sulphonyl]amino]phenyl]-5-benzofuranyl]methyl]-N,4-dimethyl-2-ethyl-1H-imidazole-5-carboxamide;
1-[[3-bromo-2-[2-[[(trifluoromethyl)sulphonyl]amino]phenyl]-5-benzofuranyl]methyl]-N-ethyl-4-methyl-2-propyl-1H-imidazole-5-carboxamide;
or a physiologically acceptable salt or solvate thereof.

5. A process for the preparation of a compound as claimed in any one of Claims 1 to 4 or a physiologically acceptable salt or solvate thereof which comprises:
(A) treating a compound of general formula (II)

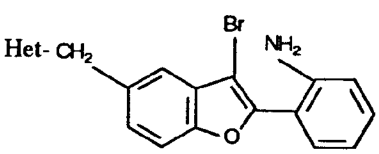

(II)

(wherein Het is as defined in general formula (I)) with trifluoromethanesulphonic anhydride or trifluoromethylsulphonyl chloride; or
(B) reacting of a compound of formula (III)

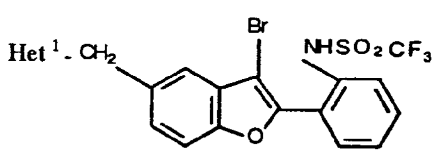

(III)

(wherein Het[1] represents a group of formula

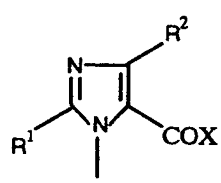

in which R[1] and R[2] are as defined in general formula (I) and X is a halogen atom (for example chlorine or bromine), or a hydroxyl or $C_{1-6}$alkoxy (for example, methoxy, ethoxy or propoxy) group) with ammonia ($R^3$ = hydrogen), methylamine ($R^3$ = methyl) or ethylamine ($R^3$ = ethyl);
followed, if necessary, by removal of any protecting group present; and when the compound of general

formula (I) is obtained as a mixture of enantiomers optionally resolving the mixture to obtain the desired enantiomer;

and/or, if desired, converting the resulting compound of general formula (I) or a salt thereof into a physiologically acceptable salt or solvate thereof.

6. A pharmaceutical composition comprising at least one compound of general formula (I) as defined in any one of Claims 1 to 4 or a physiologically acceptable salt or solvate thereof, together with at least one physiologically acceptable carrier or excipient.

7. A compound of general formula (I) as claimed in any one of Claims 1 to 4 or a physiologically acceptable salt or solvate thereof for use in therapy, for example,

(i) for use in the treatment or prophylaxis of hypertension; or

(ii) for use in the treatment or prophylaxis of congestive heart failure, acute or chronic heart failure, aortic or cardiac insufficiency, post-myocardial infarction, renal insufficiency and renal failure (for example, as a result of diabetic nephropathy, glomerular nephritis, scleroderma or renal crisis), proteinuria, Bartter's syndrome, secondary hyperaldosteronism, Reynaud's syndrome, cerebrovascular insufficiency, peripheral vascular disease, diabetic retinopathy, atherogenesis and for the improvement of vascular compliance; or

(iii) for use in the treatment or prophylaxis of cognitive disorders such as dementia (e.g. Alzheimer's disease) and other CNS disorders, such as anxiety disorders, schizophrenia, depression and alcohol or drug (e.g. cocaine) dependency; or

(iv) for use in the treatment of conditions associated with excessive or unregulated angiotensin II activity; or

(v) for use in the treatment of a condition associated with activation of the Renin-Angiotensin System.

**Claims for the following Contracting States : ES, GR**

1. A process for the preparation of compound of the general formula (I):

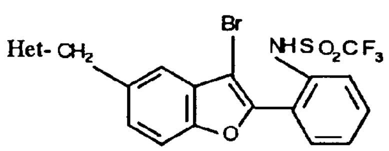

(I)

or a physiologically acceptable salt or solvate thereof wherein
Het represents an N-linked imidazolyl group of the formula

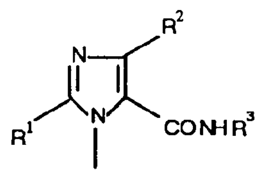

;

$R^1$ represents an ethyl or n-propyl group;
$R^2$ represents a chlorine atom or a methyl or ethyl group; and
$R^3$ represents a hydrogen atom or a methyl or ethyl group;

which comprises:

(A) treating a compound of general formula (II)

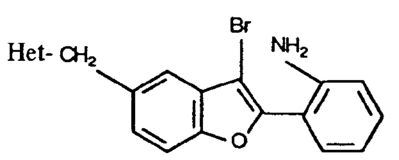

(II)

(wherein Het is as defined in general formula (I)) with trifluoromethanesulphonic anhydride or trifluoromethylsulphonyl chloride; or
(B) reacting of a compound of formula (III)

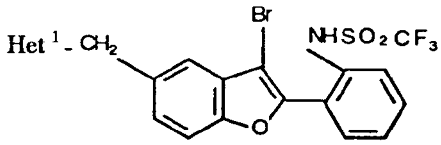

(III)

(wherein $Het^1$ represents a group of formula

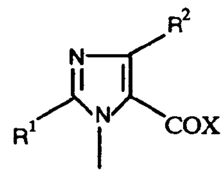

in which $R^1$ and $R^2$ are as defined in general formula (I) and X is a halogen atom (for example chlorine or bromine), or a hydroxyl or $C_{1-6}$alkoxy (for example, methoxy, ethoxy or propoxy) group) with ammonia ($R^3$ = hydrogen), methylamine ($R^3$ = methyl) or ethylamine ($R^3$ = ethyl);
followed, if necessary, by removal of any protecting group present; and when the compound of general formula (I) is obtained as a mixture of enantiomers optionally resolving the mixture to obtain the desired enantiomer;
and/or, if desired, converting the resulting compound of general formula (I) or a salt thereof into a physiologically acceptable salt or solvate thereof.

**2.** A process as claimed in Claim 1 for the prpearation of a compound of general formula (I) wherein $R^2$ represents a chlorine atom or a methyl group.

**3.** A process as claimed in Claim 1 for the preparation of a compound selected from
1-[[3-bromo-2-[2-[[(trifluoromethyl)sulphonyl]amino]phenyl]-5-benzofuranyl]methyl]-N,4-dimethyl-2-ethyl-1H-imidazole-5-carboxamide;
1-[[3-bromo-2-[2-[[(trifluoromethyl)sulphonyl]amino]phenyl]-5-benzofuranyl]methyl]-4-chloro-2-ethyl-1H-imidazole-5-carboxamide;
1-[[3-bromo-2-[2-[[(trifluoromethyl)sulphonyl]amino]phenyl]-5-benzofuranyl]methyl]-4-chloro-N-methyl-2-propyl-1H-imidazole-5-carboxamide;
1-[(3-bromo-2-[2-[[(trifluoromethyl)sulphonyl]amino]phenyl]-5-benzofuranyl]methyl]-4-chloro-N-ethyl-2-propyl-1H-imidazole-5-carboxamide;
1-[[3-bromo-2-[2-[[(trifluoromethyl)sulphonyl]amino]phenyl]-5-benzofuranyl]methyl]-4-chloro-2-ethyl-N-methyl-1H-imidazole-5-carboxamide;
1-[[3-bromo-2-[2-[[(trifluoromethyl)sulphonyl]amino]phenyl]-5-benzofuranyl]methyl]-2-ethyl-4-methyl-1H-imidazole-5-carboxamide;
1-[[3-bromo-2-[2-[[(trifluoromethyl)sulphonyl]amino]phenyl]-5-benzofuranyl]methyl]-N,4-dimethyl-2-propyl-1H-imidazole-5-carboxamide;
1-[[3-bromo-2-[2-[[(trifluoromethyl)sulphonyl]amino]phenyl]-5-benzofuranyl]methyl]-4-methyl-2-propyl-1H-imidazole-5-carboxamide;
or a physiologically acceptable salt or solvate thereof.

**4.** A process as claimed in Claim 1 for the preparation of a compound selected from
1-[[3-bromo-2-[2-[[(trifluoromethyl)sulphonyl]amino]phenyl]-5-benzofuranyl]methyl]-N,4-dimethyl-2-ethyl-1H-imidazole-5-carboxamide;
1-[[3-bromo-2-[2-[[(trifluoromethyl)sulphonyl]amino]phenyl]-5-benzofuranyl]methyl]-N-ethyl-4-methyl-2-propyl-1H-imidazole-5-carboxamide;
or a physiologically acceptable salt or solvate thereof.

European Patent Office

# EUROPEAN SEARCH REPORT

Application Number

EP 92 30 4448

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-0 028 833 (TAKEDA CHEMICAL INDUSTRIES LTD.)<br>* the whole document *<br>--- | 1,5-7 | C07D405/06<br>A61K31/415 |
| D,Y | EP-A-0 028 834 (TAKEDA CHEMICAL INDUSTRIES LTD.)<br>* the whole document *<br>--- | 1,5-7 | |
| P,X | EP-A-0 434 249 (GLAXO GROUP LIMITED)<br>* page 3, line 1 - page 7, line 15; claims; examples 92,32 * | 1-7 | |
| P,Y | <br>--- | 1,5-7 | |
| A | WO-A-9 100 281 (E.I. DU PONT DE NEMOURS AND COMPANY)<br>* page 1, line 14 - page 15, line 8 *<br>* abstract *<br>----- | 1,7 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 07 JULY 1992 | PAISDOR B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)